# EUROPEAN PATENT APPLICATION

(11) **EP 2 095 819 A1**
(43) Date of publication of application: **02.09.2009**
(21) Application number: 08152079.3
(22) Date of filing: 28.02.2008
(51) Int. Cl.: A61K 31/4174, C07D 233/54, A61P 9/10

(54) **N-benzyl imidazole derivatives and their use as aldosterone synthase inhibitors**

(71) Applicant: Maastricht University, 6211 LK Maastricht (NL); STW (Stichting voor de Technische Wetenschappen), 3527 JP Utrecht (NL); Technische Universiteit Eindhoven, 5612 AZ Eindhoven (NL)
(72) Inventor: The designation of the inventor has not yet been filed
(74) Representative: Habets, Winand

(57) **Abstract**

The invention relates to the use of a N-benzyl 5-substituted imidazole derivative having the general formula I 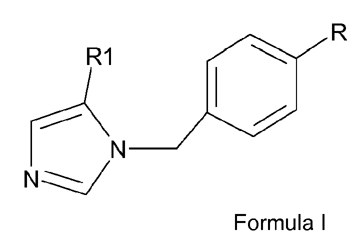 wherein R is (C₁₋₃)alkyl, (C₁₋₃)alkyloxy, halogen, nitro or cyano; R₁ is (C₁₋₆)alkyl, optionally substituted with OH, (C₁₋₃)alkyloxy, (C₁₋₃)alkylcarbonyloxy, (C₁₋₃)alkyloxycarbonyl or halogen, or (C₁₋₃)alkyloxycarbonyl; or R₁ is phenyl, optionally substituted with 1-3 substituents independently selected from (C₁₋₃)alkyl, (C₁₋₃)alkyloxy, hydroxylmethyl and halogen; or a pharmaceutically acceptable salt thereof, for the preparation of a medicament for the treatment of a disorder or disease in a subject mediated by aldosterone synthase or responsive to inhibition of aldosterone synthase.

## Description

Heart failure is a condition in which the heart is not able to cope adequately with its workload. It is characterized by a high mortality and extensive hospitalizations, and poses an increasing burden on health care (Masoudi et al. "The burden of chronic congestive heart failure in older persons: magnitude and implications for policy and research" Heart Fail Rev 2002;7:9-16). Although introduction of blockers of the renin-angiotensin system (RAS) created a breakthrough in the treatment of heart failure, death and hospitalization numbers are still high, and better therapeutic options desirable (Mann et al. "New therapeutics for chronic heart failure" Annu Rev Med 2002, 53, 59-74). More recently, clinical studies have provided evidence for a key role of the adrenal steroid hormone aldosterone in heart failure and a high therapeutic benefit of blocking its action, even if patients are already being treated by conventional medication as described above.

Although at present it is not fully clear by which mechanisms aldosterone exerts its detrimental effects, it is likely that these extend beyond the well known action of aldosterone on renal salt-handling and blood pressure. In the heart, aldosterone produces ventricular hypertrophy and extensive collagen deposition (fibrosis) in the perivascular and interstitial space. Excessive collagen deposition impairs heart function by increasing ventricular stiffness and decreasing myocardial perfusion and also causes electrical conductance dispersion, which may contribute to arrhythmias. Indeed, the ability to prevent and reverse cardiac fibrosis determines the therapeutic benefit of mineralocorticoid receptor antagonists. Only in heart failure patients with high cardiac collagen deposition, mineralocorticoid receptor antagonists are able to improve clinical outcome, whereas the extent by which cardiac collagen levels are reduced predicts patient prognosis.

The fibrosis by aldosterone is proceeded by an inflammatory process, involving macrophage and monocyte infiltration, production of chemokines, free radicals and free radical generating enzymes, and activation of the transcription factor NFκB, resulting in myocyte death and (coronary) vascular lesions. The proinflammatory and fibrotic actions of aldosterone are exacerbated by high salt intake and can be prevented and reversed by mineralocorticoid antagonists.

Other effects of aldosterone that may affect cardiac structure, cardiac electrical conductance properties and heart function involve the induction of endothelial dysfunction and decreased nitric oxide bioavailability, augmentation of angiotensin II, endothelin-1 and catecholamine actions, a reduction of baroreflex sensitivity and heart rate variability, and changed potassium and magnesium levels.
The classical approach to block aldosterone action was by using mineralocorticoid receptor antagonists. Use of these antagonist may suffer from serious sex-steroid related side effects, while in addition aldosterone levels can increase considerably. Aldosterone, next to inducing changes in the gene transcription via the mineralocorticoid receptor, exerts rapid non-genomic effects, that are likely to contribute to the pathological effects of aldosterone (Chai and Danser "Why are mineralocorticoid receptor antagonists cardioprotective?" Naunyn Schmiedebergs Arch Pharmacol 2006;374:153-62). As these effects can not be fully explained by interaction with the mineralocorticoid receptor (Haseroth et al. "Rapid nongenomic effects of aldosterone in mineralocorticoid-receptor-knockout mice" Biochem Biophys Res Commun 1999;266:257-61) it is anticipated that they are not blocked by mineralocorticoid receptor antagonists.

An alternative way to block aldosterone action is by reducing the levels of the steroid hormone itself, i.e. by inhibiting the biosynthesis of aldosterone. Aldosterone synthase (CYP11B2) is a mitochondrial cytochrome P450 enzyme which catalyses the last steps of aldosterone production in the adrenal cortex, i.e. the conversion of 11-deoxycorticosterone to aldosterone. The use of inhibitors of aldosterone synthase for the treatment of congestive heart failure and myocardial fibrosis has been suggested by Hartmann et al.( "Discovery of selective CYP11B2 inhibitors for the therapy of congestive heart failure and myocardial fibrosis.", Eur. J. Med. Chem. 2003, 38, 363-366). Such CYP11B2 inhibitors should preferably be selective with regard to the inhibition of the enzyme steroid 11β-hydroxylase (CYP11B1). This CYP11B1 enzyme has 93% sequence homology with CYP11B2 and catalyses the final steps in the biosynthesis of the glucocorticoids cortisol and cortisone.
The racemic compound fadrozole, 4-(5,6,7,8-tetrahydroimidazo[1,5-a]-pyridine-5-yl)benzonitril, is an antineoplastic agent known as an inhibitor of aromatase (CYP19), another enzyme of the cytochrome P450 family. Recently it has been reported in the International Patent Application WO 2001076574 (Novartis) that the R-enantiomer of fadrozole is an aldosterone synthase inhibitor useful in the treatment of conditions such as hypertension, congestive heart failure and others.
Further imidazo[1,5-a]pyridine derivatives have been disclosed in the International Patent application WO 2004/046145 (NOVARTIS PHARMA Gmbh) as aldosterone synthase inhibitors useful in the treatment of hypokalemia, hypertension, congestive heart failure, renal failure, atherosclerosis, coronary heart diseases, post myocardial infarction, restenosis, increased formation of collagen, fibrosis and remodeling following hypertension, endothelial dysfunction, renal dysfunction, nephropathy, syndrome X and obesity.

Condensed imidazolo derivatives closely related in structure to fadrozole and having aldosterone synthase and aromatase activity were recently disclosed in the International Patent Application WO 2007/024945 (NOVARTIS PHARMA Gmbh). N-tetrahydroquinolin-4-yl and N-isochroman-4-yl 5-substituted imidazole derivatives were disclosed in WO 2007/117982 (Novartis Pharma Gmbh) as inhibitors of CYP11B2 and CYP11B1.
Nevertheless, a need for compounds which are selective inhibitors of aldosterone synthase remains.

To this aim the present invention provides N-benzyl 5-substituted imidazole derivatives having the general formula I 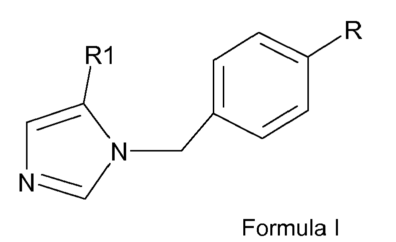 wherein
R is (C₁₋₃)alkyl, (C₁₋₃)alkyloxy, halogen, nitro or cyano;
R₁ is (C₁₋₆)alkyl, optionally substituted with OH, (C₁₋₃)alkyloxy, (C₁₋₃)alkylcarbonyloxy, (C₁₋₃)alkyloxycarbonyl or halogen, or (C₁₋₃)alkyloxycarbonyl; or
R₁ is phenyl, optionally substituted with 1-3 substituents independently selected from (C₁₋₃)alkyl, (C₁₋₃)alkyloxy, hydroxymethyl and halogen; or a pharmaceutically acceptable salt thereof, for the preparation of a medicament for the treatment of a disorder or disease in a subject mediated by aldosterone synthase or responsive to inhibition of aldosterone synthase, such as in the treatment of congestive heart failure, post-myocardial infarction, hypertension, hypokalema, nephropathy, renal failure, hyperaldosteronemia, fibrosis or remodeling following hypertension and endothelial dysfunction.

The aldosterone synthase inhibitors of the present invention are distinguished from the prior art inhibitors by the absence of a fused bicyclic ring system and by the presence of a structurally simple 5-substituent at the imidazole ring while retaining potent and selective inhibitory activity for the CYP11B2 enzyme.

The term (C₁₋₃)alkyl as used in the definition of Formula I means a branched or unbranched alkyl group having 1-3 carbon atoms, like propyl, isopropyl, ethyl and methyl.
The term (C₁₋₆)alkyl likewise means a branched or unbranched alkyl group having 1-6 carbon atoms, like hexyl, pentyl, butyl, isobutyl, tertiary butyl, propyl, isopropyl, ethyl and methyl.
In the terms (C₁₋₃)alkyloxy, (C₁₋₃)alkylcarbonyloxy and (C₁₋₃)oxycarbonyl, (C₁₋₃)alkyl has the meaning as defined above.
The term halogen means F, Cl, Br or I.

In one aspect the invention relates to the use of N-benzyl 5-substituted imidazole derivatives having the general formula I in the preparation of a medicament for the treatment of congestive heart failure and myocardial fibrosis.

There is a preference for the use in the invention of N-benzyl 5-substituted imidazole derivatives having the general formula I wherein R is (C₁₋₃)alkyl, (C₁₋₃)alkyloxy, halogen, nitro or cyano, and R₁ is phenyl, optionally substituted with 1-3 substituents independently selected from (C₁₋₃)alkyl, (C₁₋₃)alkyloxy, hydroxymethyl and halogen. Further preferred are the compounds of formula I wherein R is cyano and R₁ is phenyl, optionally substituted with halogen.

Specifically preferred N-benzyl 5-substituted imidazole derivatives for use in the invention are:
- 3-[1-(4-fluorobenzyl)-1*H*-imidazol-5-yl]propanol;
- methyl 3-[1-(4-cyanobenzyl)-1*H*-imidazol-5-yl]propanoate ;
- methyl 3-[1-(4-bromobenzyl)-1*H*-imidazol-5-yl]propanoate;
- 1-(4-cyanobenzyl)-5-hydroxymethyl-1*H*-imidazole;
- 4-[(5-methyl-1*H*-imidazol-1-yl)methyl]benzonitrile;
- 4-[(5-phenyl-1*H*-imidazol-1-yl)methyl]benzonitrile;
- 4-[(5-formyl-1*H*-imidazol-1-yl)methyl]benzonitrile;
- methyl 1-(4-cyanobenzyl)-1*H*-imidazole-5-carboxylate;
- 1-(4-nitrobenzyl)-5-phenyl-1*H*-imidazole;
- 4-[(5-(2-methylphenyl)-1*H*-imidazol-1-yl)methyl]benzonitrile; and
- 4-[(5-(3-fluorophenyl)-1*H*-imidazol-1-yl)methyl]benzonitrile; or a pharmaceutically acceptable salt thereof.

From the preferred N-benzyl 5-substituted imidazole derivatives for use in the invention methyl 3-[1-(4-cyanobenzyl)-1*H*-imidazol-5-yl]propanoate is disclosed as a synthetic intermediate in WO 2001/009128 (Yuhan Corporation), while 1-(4-cyanobenzyl)-5-hydroxymethyl-1*H*-imidazole, 4-[(5-formyl-1*H*-imidazol-1-yl)methyl]benzonitrile and methyl 1-(4-cyanobenzyl)-1*H*-imidazole-5-carboxylate were likewise disclosed as synthetic intermediates in WO 1997/27752. 4-[(5-Methyl-1*H*-imidazol-1-yl)methyl]benzonitrile was disclosed in US 4,937,250 (Ciba-Geigy Corporation) as a compound having aromatase inhibitory activity, useful for the treatment of estrogen dependent conditions.

Another aspect of the invention relates to the following novel N-benzyl 5-substituted imidazole derivatives per se:
- 3-[1-(4-fluorobenzyl)-1*H*-imidazol-5-yl]propanol;
- methyl 3-[1-(4-bromobenzyl)-1*H*-imidazol-5-yl]propanoate :

- 4-[(5-phenyl-1*H*-imidazol-1-yl)methyl]benzonitrile;
- 1-(4-nitrobenzyl)-5-phenyl-1*H*-imidazole;
- 4-[(5-(2-methylphenyl)-1*H*-imidazol-1-yl)methyl]benzonitrile; and
- 4-[(5-(3-fluorophenyl)-1*H*-imidazol-1-yl)methyl]benzonitrile; or a pharmaceutically acceptable salt thereof.

The N-benzyl 5-substituted imidazole derivatives of the invention may be prepared by methods well known in the art of organic chemistry in general.
N-Benzyl 5-substituted imidazole derivatives of Formula I can for instance be prepared starting from imidazole derivatives of Formula II, wherein R₁ has the meaning as previously defined. Following tritylation of the imidazol group the resulting compound of Formula III can be alkylated by a compound of Formula IV, wherein R has the meaning as previously defined and wherein L represents a leaving group such as a halogen. 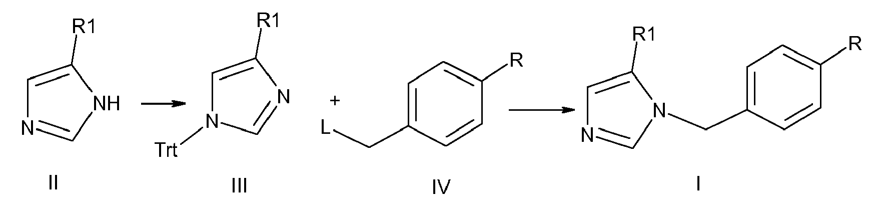 N-Benzyl 5-substituted imidazole derivatives of Formula I wherein R₁ is phenyl (represented by Formula VII), can in an alternative method be prepared by a Suzuki coupling reaction between a boronic acid derivative of Formula V, wherein R₂ represents 1-3 substituents independently selected from (C₁₋₃)alkyl, (C₁₋₃)alkyloxy, hydroxymethyl and halogen, and a brominated N-benzyl imidazole derivative of Formula VI 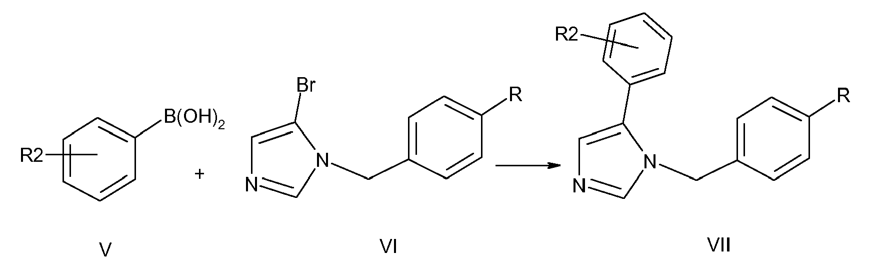 Compounds of Formula II, IV, V and VI are either commercially available or can be prepared using literature procedures.

The N-benzyl 5-substituted imidazole derivatives of Formula I and their salts may contain at least one centre of chirality, and exist therefore as stereoisomers, including enantiomers and diastereomers. The present invention includes the aforementioned stereoisomers within its scope and each of the individual R and S enantiomers of the compounds of Formula I and their salts, substantially free, i.e. associated with less than 5%, preferably less than 2%, in particular less than 1 % of the other enantiomer, and mixtures of such enantiomers in any proportions including racemic mixtures containing equal amounts of the two enantiomers.
Methods for asymmetric synthesis or chiral separation whereby the pure stereoisomers are obtained are well known in the art, e.g. synthesis with chiral induction or starting from commercially available chiral substrates, or separation of stereoisomers, for example using chromatography on chiral media or by crystallisation with a chiral counter-ion.

Pharmaceutically acceptable salts may be obtained by treating a free base of a compound of Formula I with a mineral acid such as hydrochloric acid, hydrobromic acid, phosphoric acid and sulfuric acid, or an organic acid such as for example ascorbic acid, citric acid, tartaric acid, lactic acid, maleic acid, malonic acid, fumaric acid, glycolic acid, succinic acid, propionic acid, acetic acid and methane sulfonic acid.

The compounds of the invention may exist in unsolvated as well as in solvated forms with pharmaceutically acceptable solvents such as water, ethanol and the like. In general, the solvated forms are considered equivalent to the unsolvated forms for the purpose of the invention.

The present invention further provides pharmaceutical compositions comprising a N-benzyl 5-substituted imidazole derivative having the general formula I 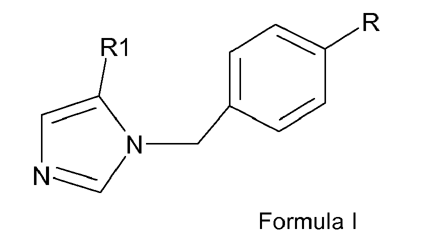 wherein
R is (C₁₋₃)alkyl, (C₁₋₃)alkyloxy, halogen, nitro or cyano;
R₁ is (C₁₋₆)alkyl, optionally substituted with OH, (C₁₋₃)alkyloxy, (C₁₋₃)alkylcarbonyloxy, (C₁₋₃)alkyloxycarbonyl or halogen, or (C₁₋₃)alkyloxycarbonyl; or
R₁ is phenyl, optionally substituted with 1-3 substituents independently selected from (C₁₋₃)alkyl, (C₁₋₃)alkyloxy, hydroxymethyl and halogen; or a pharmaceutically acceptable salt thereof, in admixture with pharmaceutically acceptable auxiliaries, with the proviso that compounds of formula I wherein R is cyano and R₁ is unsubstituted (C₁₋₆)alkyl are excluded.
The proviso relates to the disclosure of compounds having aromatase inhibitory activity in US 4,937,250 (Ciba-Geigy Corporation; supra).

The term "acceptable" means being compatible with the other ingredients of the composition and not deleterious to the recipients thereof. Compositions include e.g. those suitable for oral, sublingual, subcutaneous, intravenous, epidural, intrathecal, intramuscular, transdermal, pulmonary, local, or rectal administration, and the like, all in unit dosage forms for administration. A preferred route of administration is the oral route.

For oral administration, the active ingredient may be presented as discrete units, such as tablets, capsules, powders, granulates, solutions, suspensions, and the like. For parenteral administration, the pharmaceutical composition of the invention may be presented in unit-dose or multi-dose containers, e.g. injection liquids in predetermined amounts, for example in sealed vials and ampoules, and may also be stored in a freeze dried (lyophilized) condition requiring only the addition of sterile liquid carrier, e.g. water, prior to use.
Mixed with such pharmaceutically acceptable auxiliaries, e.g. as described in the standard reference, Gennaro, A.R. et al, Remington: The Science and Practice of Pharmacy (20th Edition, Lippincott Williams & Wilkins, 2000, see especially Part 5: Pharmaceutical Manufacturing), the active agent may be compressed into solid dosage units, such as pills, tablets, or be processed into capsules, suppositories or patches. By means of pharmaceutically acceptable liquids the active agent can be applied as a fluid composition, e.g. as an injection preparation, in the form of a solution, suspension, emulsion, or as a spray, e.g. a nasal spray.
For making solid dosage units, the use of conventional additives such as fillers, colorants, polymeric binders and the like is contemplated. In general any pharmaceutically acceptable additive which does not interfere with the function of the active compounds can be used. Suitable carriers with which the active agent of the invention can be administered as solid compositions include lactose, starch, cellulose derivatives and the like, or mixtures thereof, used in suitable amounts. For parenteral administration, aqueous suspensions, isotonic saline solutions and sterile injectable solutions may be used, containing pharmaceutically acceptable dispersing agents and/or wetting agents, such as propylene glycol or butylene glycol.
The invention further includes a pharmaceutical composition, as hereinbefore described, in combination with packaging material suitable for said composition, said packaging material including instructions for the use of the composition for the use as hereinbefore described.

The N-benzyl 5-substituted imidazole derivatives of the invention were found to be inhibitors of aldosterone synthase (CYP11B2), as determined in V79 cells stably (over)expressing human CYP11B2. Moreover the compounds were found to be selective CYP11B2 inhibitors as compared to their inhibiting activity of the enzyme 11-β-hydroxylase (CYP11B1), as determined in V79 cells stably (over)expressing human CYP11B1.

It has become evident that, in addition to the use of CYP11 B2 inhibitors in the treatment of congestive heart failure,myocardial fibrosis and after myocardial infarction, blocking the action of aldosterone is also of substantial therapeutic merit in the treatment of hypertension (particularly therapy resistant hypertension) and in kidney disease or nephropathy, in particular in case of diabetes related nephropathy. Indeed, in the treatment of these conditions, salutary effects for mineralocorticoid receptor antagonists have been reported. It is therefore expected that the aldosterone synthase inhibitors of the present invention will also be beneficial in these diseases.

Similarly, it is anticipated that inhibitors of the biosynthesis of aldosterone possess potential therapeutic merit in diseases in which aldosterone is known to play a role (and in which mineralocorticoid receptor antagonists often have been shown to be useful) as e.g.: hyperaldosteronism, edema (e.g. due to liver cirrhosis, congestive heart failure or sepsis), hypernatremia or hypervolemia or other situations in which the use of diuretics is desirable (mineralocorticoid receptor antagonists are potassium sparing diuretics), hypokalemia, fibrotic diseases, (cardiac) arrhythmias and cardiac remodeling following myocardial infarction or cardiac ischemia.

The CYP11B2 inhibitors of the invention would thus potentially be useful in the treatment of chronic heart failure, fibrosis, such as cardiac or myocardial fibrosis, hypertension, nephropathy, renal failure, and in the treatment of further aldosterone mediated diseased such as hyperaldosteroism, edema as a result of liver cirrhosis, congestive heart failure or sepsis, hypernatremia, hypervolemia, hypokalemia, cardiac arrhythmias and cardiac remodeling following myocardial infarction or cardiac ischemia.

The compounds of the invention could also be used in conjunction with other drugs, including, but not limited to beta blocking agents, cardiac glycosides, diuretics, ACE- inhibitors, AT₁-receptor antagonists, renin inhibitors and mineralocorticoid receptor antagonists.

The compounds of the invention may be administered to humans in a sufficient amount and for a sufficient amount of time to alleviate the symptoms. Illustratively, dosage levels for humans can be in the range of 0.001-50 mg per kg body weight, preferably in a dosage of 0.01-20 mg per kg body weight.

The invention is illustrated by the following Examples.

### General Methods.

### Apparatus and materials

Molecular characterization of the prepared compounds was performed by NMR spectroscopy using a Varian Mercury Vx 400 MHz or a Varian Gemini 300 MHz spectrometer, where spectra were recorded at a temperature of 298 K, and by LC-MS using a Finnigan LCQ Decca XP Max ESI-MS spectrometer and applying an Alltima HP C18 50mm, 3 µm column with an acetonitrile/water eluent gradient, and 0.1% formic acid in the eluent for ionization. Simultaneous with MS-detection, photo diode array (PDA) detection was performed. In some cases GC-MS was used to characterize the molecules, using a Shimadzu GCMS-QP5000 spectrometer applying a Zebron ZB-5 15m, 0.1 µm or a Zebron ZB-35 30m, 0.25 µm capillary column in a Shimadzu GC-17A chromatograph. A single peak for the desired product was observed in the chromatograms for all analyzed products, in either LC-MS or GC-MS.
Compounds **8-11, 16-17, 28-33, 41** and **53-56** have been acquired from commercial sources. All further reagents, catalysts, chemicals, materials and solvents were also obtained commercially and were used without further purification. When appropriate, drying of solvents was usually done by applying molsieves that were dried in an oven at 500 °C.

### Example 1

**Methyl 3-(1*H*-imidazol-4-yl)acrylate (3), methyl 3-(1*H*-imidazol-4-yl)propanoate (4), Methyl 3-(1-trityl-1*H*-imidazol-4-yl)propanoate (5) and 3-(1-(trityl)-1H-**imidazol-4-yl)propanol **(6):** These precursors were synthesized as shown in Scheme 1 starting with urocanic acid **2** by using procedures described by Stark et al., J. Med. Chem., 1996, 39 (6), 1220-1226.

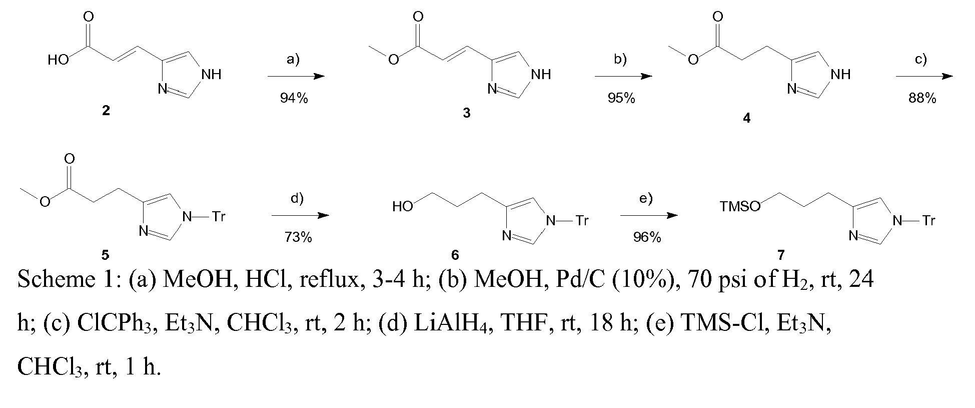

**4-[3-((Trimethylsilyl)oxy)propyl]-1-trityl-1*H*-imidazole (7):** A solution of trimethylsilyl chloride (36.0 g, 331 mmol) in 200 mL of chloroform was added drop wise to an ice-cooled solution of 3-(1-(trityl)-1H-imidazol-4-yl)propanol **6** (101.8 g, 276 mmol) and triethylamine (39.0 g, 386 mmol) in 600 mL of chloroform. After stirring for 1 hour the solution was concentrated and diluted with 600 mL of diethyl ether, giving a white precipitate. The precipitate was collected by filtration and washed with 600 mL of diethyl ether. Concentration of the filtrate and co-evaporation with diethyl ether yielded a quantitative yield of a slightly yellow solid. ¹H-NMR (CDCl₃) δ 7.35 (m, 10H), 7.15 (m, 6H), 6.55 (s, 1H), 3.61 (t, 2H), 2.60 (t, 2H), 1.85 (m, 2H), 0.10 (s, 9H). LC-MS: m/z = 369 and 243 for the TMS-deprotected H⁺-species and the trityl radical cation, the (M+1)⁺ species is not observed. GC-MS: m/z = 243 (trityl radical cation), the molecular ion is not observed.

### Example 2.

### 3-[1-(4-Cyanobenzyl)-1H-imidazol-5-yl]propanol (12)

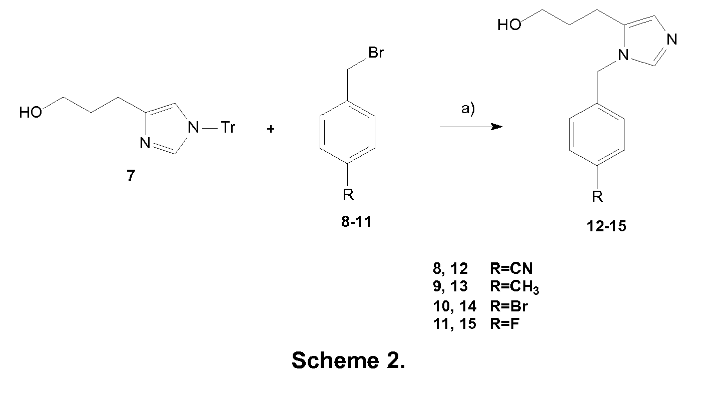

A solution of 4-(3-((trimethylsilyl)oxy)propyl)-1-trityl-1H-imidazole **7** (19.8 g, 45 mmol) and 4-cyanobenzyl bromide **8** (8.8 g, 45 mmol) in 250 mL of acetonitrile was refluxed overnight. The solution was concentrated and dissolved in a mixture of 80 mL acetic acid and 20 mL water, and this mixture was refluxed until a white solid precipitated. Water (200 mL) was added and after cooling down to room temperature the mixture was extracted twice with diethyl ether. The aqueous phase was adjusted to pH=9 with a 4M NaOH solution and was then extracted with methylene chloride. The organic extracts were dried over Na₂SO₄ and evaporated to give a slightly yellow solid. Trituration with cold acetone yielded 6.5 g (59%) of a white solid. ¹H-NMR (CDCl₃) δ 7.6 (d, 2H), 7.5 (s, 1H), 7.1 (d, 2H), 6.85 (s, 1H), 5.15 (s, 2H), 3.6 (t, 2H), 2.65 (bs, 1H), 2.5 (t, 2H), 1.8 (m, 2H). ¹³C-NMR (CDCl₃) δ 141.8, 137.4, 132.8, 131.6, 127.0, 126.9, 118.3, 112.0, 61.2, 47.8, 31.0, 20.1. LC-MS: m/z = 242 (M+1).

### Example 3

The following imidazoles were prepared (see Scheme 2) using a similar procedure as that described in Example 2.

### 3A: 3-[1-(4-Methylbenzyl)-1H-imidazol-5-yl]propanol (13):

Using 4-methylbenzyl bromide **9.** Purification was performed by column chromatography on alumina eluting with CHCl₃/MeOH (99:1 - 95:5) to give 0.9 g (87%) of a colorless oil. ¹H-NMR (CDCl₃) δ 7.41 (s, 1H), 7.13 (d, 2H), 6.95 (d, 2H), 6.80 (s, 1H), 5.00 (s, 2H), 3.61 (t, 2H), 2.60 (bs, 1H), 2.55 (t, 2H), 2.30 (s, 3H), 1.80 (m, 2H).

**3B: 3-[1-(4-Bromobenzyl)-1*H*-imidazol-5-yl]propanol (14):** Using 4-bromobenzyl bromide **10.** Purification was performed by column chromatography on alumina eluting with CHCl₃/MeOH (99:1 - 95:5) to give 0.7 g (52%) of a white solid. ¹H-NMR (CDCl₃) δ 7.45 (m, 3H), 6.90 (d, 2H), 6.81 (s, 1H), 5.02 (s, 2H), 3.61 (t, 2H), 2.50 (t, 3H), 2.1 (bs, 1H), 1.80 (m, 2H). LC-MS: m/z = 297 (M+1).

**3C: 3-[1-(4-Fluorobenzyl)-1*H*-imidazol-5-yl]propanol (15):** Using 4-fluorobenzyl bromide **11.** Purification was performed by column chromatography on silica gel eluting with CHCl₃/MeOH (95:5 - 90:10) to give 0.3 g (55%) of a white solid. ¹H-NMR (CDCl₃) δ 7.45 (s, 1H), 7.02 (d, 4H), 6.85 (s, 1H), 5.05 (s, 2H), 3.64 (t, 2H), 2.52 (t, 2H), 1.81 (m, 2H), 1.65 (bs, 1H). LC-MS: m/z = 235 (M+1).

### Example 4.

### Methyl 3-[1-(4-cyanobenzyl)-1H-imidazol-5-yl]propanoate (18).

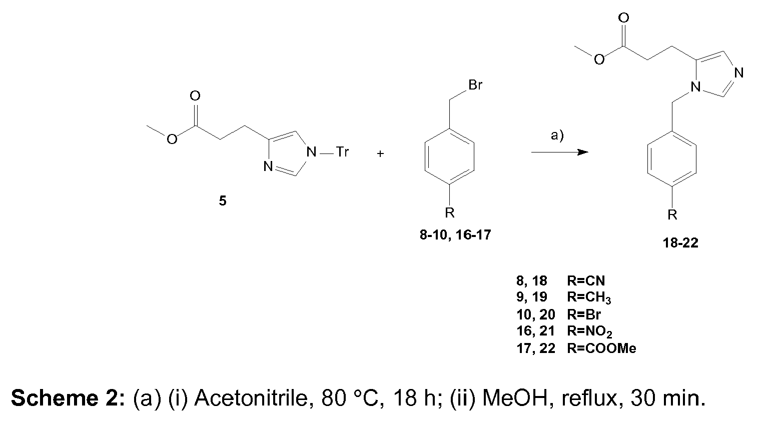

Methyl 3-(1-trityl-1*H*-imidazol-4-yl)propanoate **5** (1.0 g, 2.5 mmol) and 4-cyanobenzyl bromide **8** (0.5 g, 2.5 mmol) were dissolved in 10 mL of acetonitrile and stirred overnight at 80 °C. The mixture was concentrated and triturated using 10 mL of ethyl acetate. The precipitate was collected by filtration and washed with ethyl acetate. The solids were refluxed for 30 minutes in 20 mL of methanol. The resulting solution was evaporated in vacuo and triturated with 10 mL of ethyl acetate. After washing with ethyl acetate the solid was taken up in a saturated NaHCO₃ solution (15 mL) and this solution was extracted twice with 15 mL of chloroform. The combined organic layers were washed with a saturated NaHCO₃ solution, dried over Na₂SO₄ and evaporated to dryness giving a yellowish oil that was purified by column chromatography on silica gel eluting with CHCl₃/MeOH (95:5 - 90:10) resulting in 0.51 g (69%) of a slightly yellow solid. ¹H-NMR (CDCl₃) δ 7.62 (d, 2H), 7.5 (s, 1H), 7.10 (d, 2H), 6.9 (s, 1H), 5.19 (s, 2H), 3.63 (s, 3H), 2.69 (t, 2H), 2.59 (t, 2H). LC-MS: m/z = 270 (M+1).

### Example 5.

The following imidazoles were prepared (see Scheme 3) using a similar procedure as described in Example 2.

### 5A: Methyl 3-[1-(4-methylbenzyl)-1H-imidazol-5-yl]propanoate (19):

Using 4-methylbenzyl bromide **9** yielding 0.36 g (55%) of a white solid after extraction. ¹H-NMR (CDCl₃) δ 7.45 (s, 1H), 7.15 (d, 2H), 6.95 (d, 2H), 6.81 (s, 1H), 5.02 (s, 2H), 3.65 (s, 3H), 2.77 (t, 2H), 2.57 (t, 2H), 2.35 (s, 3H). LC-MS: m/z = 259 (M+1).

### 5B: Methyl 3-[1-(4-bromobenzyl)-1H-imidazol-5-yl]propanoate (20):

Using 4-bromobenzyl bromide **10.** Purification was performed by column chromatography on silica gel eluting with CHCl₃/MeOH (95:5) resulting in 0.49 g (60%) of a white solid. ¹H-NMR (CDCl₃) δ 7.49 (s, 1H), 7.45 (d, 2H), 6.91 (d, 2H), 6.82 (s, 1H) 5.04 (s, 2H), 3.65 (s, 3H), 2.71 (t, 2H), 2.59 (t, 2H). LC-MS: m/z = 325 (M+1).

### 5C: Methyl 3-[1-(4-nitrobenzyl)-1H-imidazol-5-yl]propanoate (21):

Using 4-nitrobenzyl bromide **16** yielding 0.57 g (78%) of a colorless oil after extraction. ¹H-NMR (CDCl₃) δ 8.20 (d, 2H), 7.51 (s, 1H), 7.18 (d, 2H), 6.88 (s, 1H), 5.22 (s, 2H), 3.63 (s, 3H), 2.70 (t, 2H), 2.61 (t, 2H). LC-MS: m/z = 290 (M+1). **5D: Methyl 4-[(5-(3-methoxy-3-oxopropyl)-1*H*-imidazol-1-yl)methyl]benzoate (22):** Using methyl 4-(bromomethyl)benzoate **17.** Purification was performed by column chromatography on silica gel eluting with CHCl₃/MeOH (95:5 - 90:10) resulting in 0.44 g (60%) of a white solid. ¹H-NMR (CDCl₃) δ 8.01 (d, 2H), 7.51 (s, 1H), 7.07 (d, 2H), 6.86 (s, 1H), 5.18 (s, 2H), 3.92 (s, 3H), 3.65 (s, 3H), 2.71 (t, 2H), 2.59 (t, 2H). LC-MS: m/z = 303 (M+1).

### Example 6.

### 4-[(5-(3-Chloropropyl)-1H-imidazol-1-yl)methyl]benzonitrile (23).

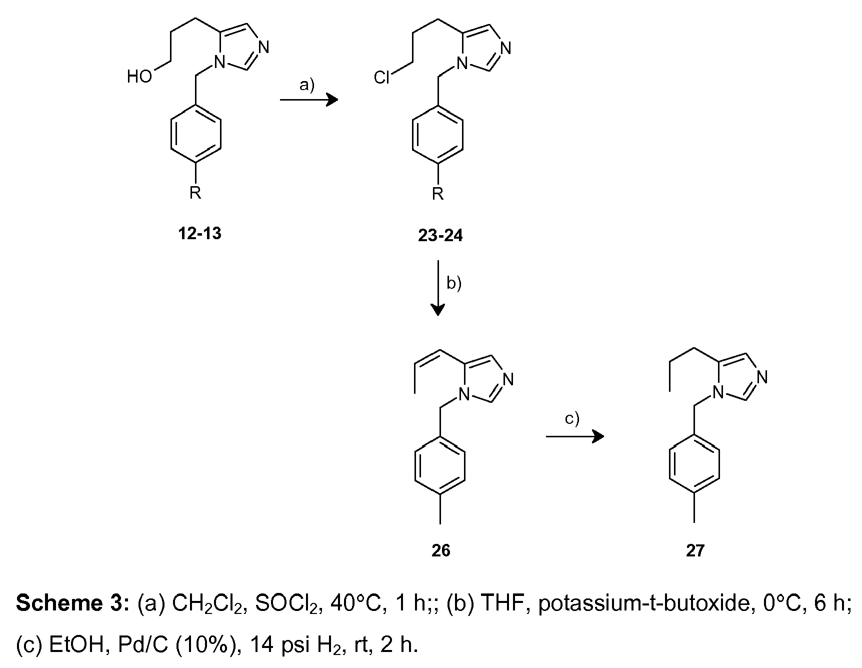

A suspension of 3-[1-(4-cyanobenzyl)-1*H*-imidazol-5-yl]propanol **12** (6.2 g, 25.8 mmol) in 50 mL of methylene chloride was added drop wise to a solution of thionyl chloride (7.8 g, 32 mmol) in 10 mL of methylene chloride. After addition the clear solution was heated at 40 °C. During heating a white solid precipitated. After 1 hour the mixture was extracted twice with 50 mL of a saturated NaHCO₃ solution. The aqueous phase was extracted back with methylene chloride. The combined organic layers were dried over Na₂SO₄ and evaporated in vacuo to yield 6.0 g (89%) of a white solid. ¹H-NMR (CDCl₃) δ 7.62 (d, 2H), 7.51 (s, 1H), 7.12 (d, 2H), 6.80 (s, 1H), 5.18 (s, 2H), 3.63 (t, 2H), 2.59 (t, 2H), 1.99 (m, 2H). ¹³C NMR (CDCl₃) δ 141.5, 137.6, 132.7, 130.1, 127.3, 126.8, 118.1, 112.0, 47.7, 43.7, 30.8, 20.7. LC-MS: m/z = 260 (M+1).

### Example 7 (see Scheme 3).

### 5-(3-Chloropropyl)-1-(4-methylbenzyl)-1H-imidazole (24).

A similar procedure as that described for example 6 was followed, using 3-[1-(4-methylbenzyl)-1*H*-imidazol-5-yl]propanol **13** as starting compound, giving 0.9 g (88%) of a white solid. ¹H-NMR (CDCl₃) δ 7.43 (s, 1H), 7.15 (d, 2H), 6.91 (d, 2H), 6.81 (s, 1H), 5.01 (s, 2H), 3.61 (t, 2H), 2.61 (t, 2H), 2.32 (s, 3H) 1.95 (m, 2H).

### Example 8 (see Scheme 3).

### 1-(4-Methylbenzyl)-5-prop-1-enyl-1H-imidazole (26)

A solution of potassium tertbutoxide (0.77 g, 6.8 mmol) in 10 mL of freshly distilled tetrahydrofuran was added drop wise to an ice-cooled solution of 5-(3-chloropropyl)-1-(4-methylbenzyl)-1*H*-imidazole **24** (0.85 g, 3.4 mmol) in 5 mL of freshly distilled tetrahydrofuran. After 6 hours 50 mL of water was added and the mixture was brought to pH 2 with 30 mL of a 1 M HCl solution. The mixture was extracted two times with 40 mL of diethyl ether. The aqueous phase was brought to pH 8 with approximately 6 mL of a 1 M NaOH solution and was then extracted two times with 40 mL of methylene chloride. The collected organic phases were dried over Na₂SO₄ and evaporated in vacuo to yield 0.54 g of brownish oil. Purification was performed by column chromatography on silica eluting with chloroform/methanol (97.5:2.5) resulting in 0.35 g (49%) of a yellowish oil. The material is a 3:1 mixture of trans and cis isomer. ¹H-NMR (CDCl₃) δ 7.45 and 7.40 (both s, 1H), 7.13 (m, 3H), 6.95 (d, 2H), 6.05 and 5.78 (both m, 2H), 5.02 (s, 2H), 2.32 (s, 3H), 1.85 + 1.79 (both d, 3H). LC-MS: m/z = 213 (M+1). GC-MS: m/z = 212 (molecular ion).

### Example 9 (see Scheme 4).

### 1-(4-Methylbenzyl)-5-propyl-1H-imidazole (27)

1-(4-Methylbenzyl)-5-prop-1-enyl-1*H*-imidazole **26** (0.2 g, 0.9 mmol) was dissolved in 2 mL of ethanol and under an argon atmosphere 10 mg of palladium (10%) on carbon was added. After three cycles of vacuum-argon and 2 cycles of vacuum-hydrogen the mixture was stirred for 2 hours at ambient temperature with an open connection to a balloon filled with hydrogen gas. The mixture was filtered over celite, the celite was washed with ethanol, and the ethanol was evaporated in vacuo to yield 0.16 g (80%) of a white solid. ¹H-NMR (CDCl₃) δ 7.42 (s, 1H), 7.15 (d, 2H), 6.95 (d, 2H), 6.8 (s, 1H), 5.00 (s, 2H), 2.38 (t, 2H), 2.30 (s, 3H), 1.59 (m, 2H), 0.95 (t, 3H). LC-MS: m/z = 215 (M+1). GC-MS: m/z = 214 (molecular ion).

### Example 10.

### 10A: 4-Methyl-1-trityl-1H-imidazole (34).

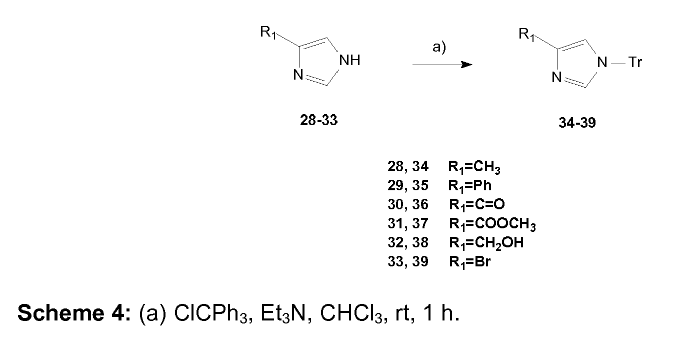

A solution of 4-methyl-1*H*-imidazole **28** (0.99 g, 12 mmol) and triethyl amine (3.4 g, 34 mmol) in 15 mL of chloroform was added to an ice-cooled solution of trityl chloride (3.7 g, 13 mmol) in 15 mL of chloroform. The reaction mixture was stirred for 1 hour at ambient temperature, and was then washed twice with 15 mL portions of a 0.5M HCl solution, and once with 15 mL of a saturated NaHCO₃ solution. The organic layer was dried over Na₂SO₄ and evaporated in vacuo. The resulting solid was triturated with 15 mL of diethyl ether, filtered and washed with diethyl ether yielding 3.7 g (93%) of a white solid. ¹H-NMR (CDCl₃) δ 7.32 (m, 10H), 7.15 (m, 6H), 6.51 (s, 1H), 2.10 (s, 3H). LC-MS: m/z = 325 (M+1) and 243 (trityl radical cation). GC-MS: m/z = 243 (trityl radical cation), the molecular ion is not observed.

The following imidazoles were prepared using a similar procedure as that for compound **34**:

### 10A: 4-Phenyl-1-trityl-1H-imidazole (35)

Using 4-phenyl-1*H*-imidazole **29** yielding 2.4 g (90%) of a white solid. ¹H-NMR (CDCl₃) δ 7.7 (d, 2H), 7.48 (s, 1H), 7.32 (m 11H), 7.20 (m, 7H), 7.10 (s, 1H). LC-MS: m/z = 387 (M+1) and 243 (trityl radical cation). GC-MS: m/z = 386 (molecular ion) and 243 (trityl radical cation).

### 10B: 1-Trityl-1H-imidazole-4-carbaldehyde (36)

A few mL of N,N-dimethylformamide (DMF) were used as co-solvent to dissolve the starting compound 1*H*-imidazole-4-carbaldehyde **30**. The DMF was removed by trituration with water, followed by trituration with diethyl ether to yield 0.6 g (69%) of a white solid. ¹H-NMR (CDCl₃) δ 9.85 (s, 1H), 7.61 (s, 1H), 7.52 (s, 1H), 7.35 (m, 9H), 7.10 (m, 6H). LC-MS: m/z = 677 (2M+1) and 243 (trityl radical cation). GC-MS: m/z = 243 (trityl radical cation).

### 10C: Methyl 1-trityl-1H-imidazole-4-carboxylate (37)

A few mL of N,N-dimethylformamide (DMF) were used as co-solvent to dissolve the starting compound methyl 1*H*-imidazole-4-carboxylate **31.** The DMF was removed by trituration with water, followed by trituration with diethyl ether to yield 0.4 g (56%) of a white solid. ¹H-NMR (CDCl₃) δ 7.59 (s, 1H), 7.62 (s, 1H), 7.35 (m, 9H), 7.12 (m, 6H), 3.85 (s, 3H). LC-MS: m/z = 737 (2M+1) and 243 (trityl radical cation). GC-MS: m/z = 243 (trityl radical cation).

### 10D: 4-Bromo-1-trityl-1H-imidazole (39)

Using 4-bromo-*1H*-imidazole **33** yielding 0.77 g (73%) of a white solid. ¹H-NMR (CDCl₃) δ 7.35 (m, 9H), 7.30 (s, 1H), 7.10 (m, 6H), 6.80 (s, 1H). LC-MS: m/z = 391 (M+1) and 243 (trityl radical cation). GC-MS: m/z = 243 (trityl radical cation).

### Example 11 (see Scheme 5).

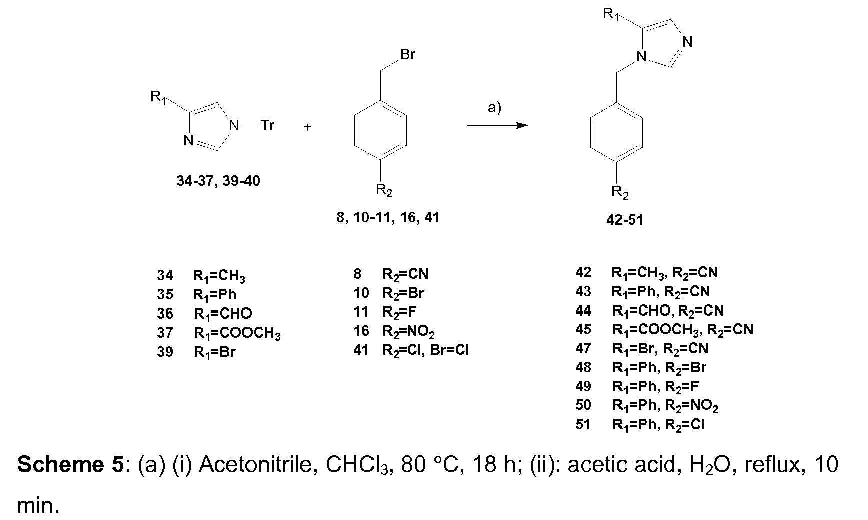

### 11A: 1-(4-Fluorobenzyl)-5-phenyl-1H-imidazole (49)

A solution of 4-phenyl-1-trityl-1*H*-imidazole **35** (0.25 g, 0.65 mmol) and 1-(bromomethyl)-4-fluorobenzene **11** (0.12 g, 0.65 mmol) in 2 mL of acetonitrile and 0.5 mL of chloroform was stirred overnight at 80 °C. The solution was concentrated and dissolved in a mixture of 1.25 mL acetic acid and 1 mL of water, and this mixture was refluxed until a white solid precipitated. A 1 M HCl solution (7.5 mL) was added and after cooling down to room temperature the mixture was extracted twice with 5 mL portions of diethyl ether. The organic phase was extracted with 7.5 mL of a 1 M solution of HCl. The combined aqueous phases were adjusted to pH 8 with a 6M NaOH solution and were then extracted twice with 10 mL portions of methylene chloride. The methylene chloride extracts were dried over Na₂SO₄, and evaporated in vacuo to give a slightly yellow oil. This crude product was purified by column chromatography using silica gel and eluting with a chloroform/methanol gradient yielding 60 mg (37%) of a colorless oil. ¹H-NMR (CDCl₃) δ 7.56 (s, 1H), 7.35 (m, 3H), 7.25 (d, 2H), 7.12 (s, 1H), 6.95 (d, 4H), 6.95 (s, 1H), 5.13 (s, 2H). LC-MS: m/z = 253 (M+1).

The following imidazoles were prepared using a similar procedure as that for compound **49:**

### 11B: 4-[(5-Methyl-1H-imidazol-1-yl)methyl]benzonitrile (42)

Using 4-methyl-1-trityl-1*H*-imidazole **34** yielding 0.71 g (78%) of a slightly yellow solid. ¹H-NMR (CDCl₃) δ 7.65 (d, 2H), 7.5 (s, 1H), 7.11 (d, 2H), 6.9 (s, 1H), 5.14 (s, 2H), 2.05 (s, 3H). LC-MS: m/z = 198 (M+1). GC-MS: m/z = 197 (molecular ion).

### 11C: 4-[(5-Phenyl-1H-imidazol-1-yl)methyl]benzonitrile (43)

Using 4-phenyl-1-trityl-1*H*-imidazole **35** yielding 5.5 g (68%) of white solid. ¹H-NMR (CDCl₃) δ 7.6 (m, 3H), 7.35 (m, 3H), 7.2 (m, 2H), 7.15 (s, 1H), 7.05 (d, 2H), 5.2 (s, 2H). ¹³C NMR δ 142.1, 138.6, 133.4, 132.7, 129.2, 128.8 (2x), 128.4, 127.1, 118.3, 112.0, 48.3. LC-MS: m/z = 260 (M+1). GC-MS: m/z = 259 (molecular ion).

### 11D: 4-[(5-Formyl-1H-imidazol-1-yl)methyl]benzonitrile (44)

Using 1-trityl-1*H*-imidazole-4-carbaldehyde 36 yielding 57 mg (37%) of a slightly yellow solid. ¹H-NMR (CDCl₃) δ 9.75 (s, 1H), 7.9 (s, 1H), 7.75 (s, 1H), 7.61 (d, 2H), 7.25 (d, 2H), 5.59 (s, 2H). LC-MS: m/z = 212 (M+1).

### 11E: Methyl 1-(4-cyanobenzyl)-1H-imidazole-5-carboxylate (45)

Using methyl 1-trityl-1*H*-imidazole-4-carboxylate 37 yielding 27 mg (11%) of a white solid. ¹H-NMR (CDCl₃) δ 7.8 (s, 1H), 7.69 (s, 1H), 7.61 (d, 2H), 7.20 (d, 2H), 5.59 (s, 2H), 3.80 (s, 3H). LC-MS: m/z = 242 (M+1).

### 11F: 4-[(5-Bromo-1H-imidazol-1-yl)methyl]benzonitrile (47)

Using 4-bromo-1-trityl-1*H*-imidazole **39** yielding 84 mg (31%) of a white solid. ¹H-NMR (CDCl₃) δ 7.65 (s, 1H), 7.62 (d, 2H), 7.20 (d, 2H), 7.10 (s, 1H), 5.21 (s, 2H). LC-MS: m/z = 262 (M+1). GC-MS: m/z = 261 and 263 (ionic isotopes).

### 11G: 1-(4-Bromobenzyl)-5-phenyl-1H-imidazole (48)

Using 4-phenyl-1-trityl-1*H*-imidazole **35** and 4-bromobenzyl bromide **10** yielding 79 mg (39%) of a slightly yellow solid. ¹H-NMR (CDCl₃) δ 7.58 (s, 1H), 7.40 (d, 2H), 7.36 (m, 3H), 7.26 (m, 2H), 7.15 (s, 1H), 6.85 (d, 2H), 5.1 (s, 2H). LC-MS: m/z = 315 (M+1).

### 11H: 1-(4-Nitrobenzyl)-5-phenyl-1H-imidazole (50)

Using 4-phenyl-1-trityl-1*H*-imidazole **35** and 4-nitrobenzyl bromide **16** yielding 72 mg (40%) of a yellowish solid. ¹H-NMR (CDCl₃) δ 8.15 (d, 2H), 7.65 (s, 1H), 7.35 (m, 3H), 7.26 (m, 2H), 7.17 (s, 1H), 7.10 (d, 2H), 5.27 (s, 2H). LC-MS: m/z = 280 (M+1).

### 11I: 1-(4-Chlorobenzyl)-5-phenyl-1H-imidazole (51)

Using 4-phenyl-1-trityl-1*H*-imidazole **35** and 4-chlorobenzyl chloride **41.** For speeding up the reaction, ca. 1 molar equivalent of Nal was added to the reaction mixture to convert the benzyl chloride to the benzyl iodide. Purification yielded 21 mg (12%) of a yellowish oil. ¹H-NMR (CDCl₃) δ 7.58 (s, 1H), 7.35 (m, 3H), 7.25 (m, 4H), 7.14 (s, 1H), 6.91 (d, 2H), 5.12 (s, 2H). LC-MS: m/z = 269 and 271 (M+1 isotopic ions).

### Example 12.

### 12A: 4-[(5-(2-Methylphenyl)-1H-imidazol-1-yl)methyl]benzonitrile (57

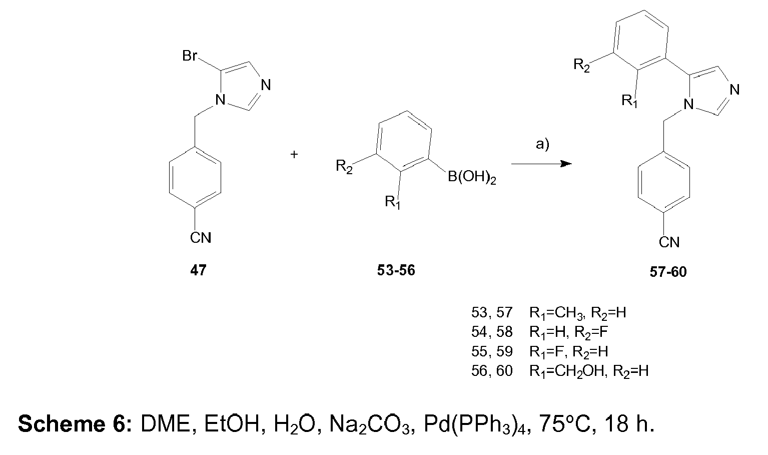

4-[(5-Bromo-1*H*-imidazol-1-yl)methyl]benzonitrile **47** (80 mg, 0.31 mmol), 2-methylphenylboronic acid **53** (46 mg, 0.34 mmol) and Na₂CO₃ (0.76 mmol) were weighed in a Schlenck tube, capped with a rubber septum. The reaction tube was filled with argon and subsequently with 1.5 mL of a 1:1:1 mixture of water, dimethoxy ethane and ethanol. The mixture was heated to 75 °C upon which a clear solution developed. Three cycles of freeze-pump-thaw were performed to remove the oxygen from the reaction mixture, and tetrakis(triphenylphosphine)palladium(0) (0.7 mg, 0.6 µmol) was added. The reaction mixture was then stirred overnight at 75 °C. The mixture was concentrated, 2.5 mL of chloroform was added, and the mixture was washed twice with 2 mL portions of a saturated NaHCO₃ solution. The organic layer was dried over Na₂SO₄ and the solvent was evaporated in vacuo giving crude product that was purified by column chromatography over silica eluting with chloroform/methanol (98:2) yielding 46 mg (55%) of a colorless oil. ¹H-NMR (CDCl₃) δ 7.67 (s, 1H), 7.51 (d, 2H), 7.3-7.1 (m, 3H), 7.04 (m, 2H), 6.95 (d, 2H), 4.98 (s, 2H), 2.01 (s, 3H). LC-MS: m/z = 274 (M+1).

The following imidazoles were prepared using a similar procedure as that for compound **57:**

### 12B: 4-[(5-(3-Fluorophenyl)-1H-imidazol-1-yl)methyl]benzonitrile (58):

Using 3-fluorophenylboronic acid **54** and purifying by column chromatography over silica eluting with ethyl acetate/hexane (2:1 - 4:1) yielded 52 mg (61%) of a white solid. ¹H-NMR (CDCl₃) δ 7.63 (s, 1H), 7.58 (d, 2H), 7.33 (m, 1H), 7.20 (s, 1H), 7.09 (d, 2H), 7.03 (m, 2H), 6.94 (d, 1H), 5.24 (s, 2H). LC-MS: m/z = 278 (M+1).

### 12C: 4-[(5-(2-Fluorophenyl)-1H-imidazol-1-yl)methyl]benzonitrile (59):

Using 2-fluorophenylboronic acid 55 and purifying by column chromatography over silica eluting with ethyl acetate/hexane (2:1 - 4:1) yielded 32 mg (38%) of a slightly yellow solid. ¹H-NMR (CDCl₃) δ 7.64 (s, 1H), 7.53 (d, 2H), 7.37 (m, 1H), 7.15 (m, 4H), 7.05 (d, 2H), 5.15 (s, 2H). LC-MS: m/z = 278 (M+1).

### 12D: 4-((5-[2-(Hydroxymethyl)phenyl]-1H-imidazol-1-yl) methyl)benzonitrile (60):

Using 2,1-benzoxaborol-1(3*H*)-ol **56** and purifying by column chromatography over silica eluting with chloroform/methanol (98:2 - 96:4) yielded 0.20 g (53%) of a slightly yellow solid. ¹H-NMR (CDCl₃) δ 7.62 (s, 1H), 7,52 (m, 3H), 7,42 (t, 1H), 7.26 (t, 1H), 7.06 (s, 1H), 7.02 (d, 1H), 6.97 (d, 2H), 5.02 (s, 2H), 4.39 (s, 2H). LC-MS: m/z = 290 (M+1).

### Example 13.

Inhibitor potencies for human CYP11B1 and CYP11B2 were determined in an *in vitro* assay using V79 cells in which human CYP11B1 or CYP11B2 are expressed.

The V79 cells stably (over)expressing human CYP11 B2 were developed in the laboratory of Prof. Rita Bernhardt, Institute of Biochemistry, Saarland University, Saarbrücken, Germany :
- Denner K, Doehmer J,Bernhardt R. "Cloning of CYP11B1 and CYP11B2 from normal human adrenal and their functional expression in COS-7 and V79 Chinese hamster cells" Endocr Res 1995;21:443-8;
- Bottner B, Denner K, Bernhardt R "Conferring aldosterone synthesis to human CYP11B1 by replacing key amino acid residues with CYP11B2 ones", Eur. J. Biochem. 1998; 252: 458-466;
- Denner K, Vogel R, Schmalix W, Doehmer J, Bernhardt R.: "Cloning and stable expression of the human mitochondrial cytochrome P45011B1 cDNA in V79 Chinese hamster cells and their application for testing of potential inhibitors." Pharmacogenetics 1995; 5: 89-96;
- Denner K, Bernhardt R. "Inhibition studies of steroid conversions mediated by human CYP11B1 and CYP11B2 expressed in cell cultures". In: Oxygen Homeostasis and Its Dynamics. Springer-Verlag, Tokyo, Berlin, Heidelberg, New York 1998, pp 231-236;
- Ehmer PB, Bureik M, Bernhardt R, Muller U, Hartmann RW. "Development of a test system for inhibitors of human aldosterone synthase (CYP11B2): screening in fission yeast and evaluation of selectivity in V79 cells." J. Steroid Biochem Mol Biol. 2002; 81: 173-179.

The human CYP11B1 (over)expressing V79 cells (stably transfected with a pcDNA3.1 vector, carrying a hygromycin resistance box) were constructed at Organon (Newhouse, UK) in the following manner. The full length cDNA for human CYP11B1 was obtained by PCR from human adrenal cDNA as described by Kawamoto et al ("Cloning of cDNA and genomic DNA for human cytochrome P-45011 beta" FEBS Lett 1990;269:345-9) and cloned into pPCR SCRIPT (Stratagene, La Jolla, USA). After digestion with Xho I and Not I, the obtained cDNA was inserted into a Xho I/Not I digested pcDNA3.1 vector (InVitrogen, Breda, Netherlands), carrying a hygromycin resistance box. Following transfection to the V79 cells, positive cells were selected based on their hygromycin resistance. The presence of the CYP11B1 gene was confirmed by PCR and the presence of 11β-hydroxylase activity (i.e. the ability to produce corticosterone or cortisol from 11-deoxycorticosterone or 11-deoxycortisol respectively). Although no electron-transporting proteins were co-transfected, the CYP11B1 expressing V79 cells showed abundant 11β-hydroxylase activity, as was previously also shown by Denner et al. for both CYP11B1 and CYP11 B2 [1]. In normal (non transfected) V79 cells, no detectable 11β-hydroxylase activity or PCR signals were found.

Cells were cultured under standard conditions in DMEM/FK12 medium (Gibco, Gaitersburg, USA) supplemented with 10% foetal calf serum (Hyclone, Logan, USA), penicillin/streptomycin (100 U/ml and 100 µg/ml, respectively Gibco). Cell culturing and incubation experiments (see below) with the cells were performed at 37°C in a humid environment and 5%CO2 atmosphere.

For assessing inhibitor potencies, cells were transferred to 12 well plates and grown until they were confluent. Next, cells were incubated for 1 hour in serum free medium with cumulative inhibitor concentrations. Subsequently, 11-deoxycorticosterone (Sigma-Aldrich, St Louis, USA) was added as substrate to obtain a final concentration of 500 nM (0.5 times Km), following incubation of the plates for another hour for V79 CYP11B1 or another three hours for V79 CYP11B2. After these incubation times, medium was collected for steroid analysis by HPLC. To extract the (produced) steroids from the medium, 1 ml aliquots of medium were mixed with 1 ml 1 M sodium-glycine buffer (pH 10.5) containing 500 nM methylprednisolone (Sigma-Aldrich) as internal standard and 5 ml of diethylether (Biosolve, Valkenswaard, the Netherlands). After shaking for 3 minutes, the samples were briefly centrifuged and the organic phase (containing the steroids) transferred into another tube and blown to dryness under a stream of nitrogen. The dried extracts were dissolved in 100 µl of mobile phase to be used for automated HPLC analysis. The HPLC system comprised a MR column (4.6 x 50 mm, particle size 2.5 µm; Shimadzu, Tokyo, Japan) as a stationary phase and a mixture of 680/320/1 (v/v/v) milliQ water, acetonitrile and trifluoro-acetic acid as mobile phase at a flow rate of 1.0 ml/minute. Steroids were detected by UV absorption at 243 nm (Shimadzu, Tokyo, Japan). To quantitate the steroids, peak area ratios versus the internal standard (methylprednisolone) were determined and compared with calibration curves.

Determination of IC₅₀ values (potencies) was based on at least 5 inhibitor concentrations and was derived from the generic equation for enzyme inhibition at fixed substrate concentration vᵢ = v₀/(1 + I/IC50) with vᵢ and v₀ being the reaction velocities in the presence or absence of inhibitor respectively, and I being the inhibitor concentration (Cornish-Bowden A, "*Analysis of enzyme kinetic data*.", Oxford University Press, Oxford, 1995). All analyses were conducted at least in duplicate. IC₅₀ values for a representative number of compounds according to the invention are presented in **Table I.** The data demonstrate that the compounds have both potent and selective (ratio) inhibitory activity at the CYP11B2 receptor.

**Table I**

| COMPOUND | IC₅₀ 11B2 nM | IC₅₀ 11B1 nM | Ratio |
|---|---|---|---|
| 3-[1-(4-fluorobenzyl)-1*H*-imidazol-5-yl]propanol (**15**) | 16.8 | 115.5 | 6.9 |
| methyl 3-[1-(4-cyanobenzyl)-1*H*-imidazol-5-yl]propanoate (**18**) | 3.5 | 31.1 | 8.9 |
| methyl 3-[1-(4-bromobenzyl)-1*H*-imidazol-5-yl]propanoate (**20**) | 4.0 | 57.4 | 14.4 |
| 1-(4-cyanobenzyl)-5-hydroxymethyl-1*H*-imidazole (**52**) | 28.5 | 285.2 | 10 |
| 4-[(5-methyl-1*H*-imidazol-1-yl)methyl]benzonitrile (**42**) | 12.3 | 140.5 | 11.4 |
| 4-[(5-phenyl-1*H*-imidazol-1-yl)methyl]benzonitrile (**43**) | 1.7 | 27.5 | 15.7 |
| 4-[(5-formyl-1*H*-imidazol-1-yl)methyl]benzonitrile (**44**) | 61.8 | 478 | 7.7 |
| methyl 1-(4-cyanobenzyl)-1*H*-imidazole-5-carboxylate (**45**) | 7.1 | 47.8 | 6.7 |
| 1-(4-nitrobenzyl)-5-phenyl-1*H*-imidazole (**50**) | 2.3 | 13.7 | 5.9 |
| 4-[(5-(2-methylphenyl)-1*H*-imidazol-1-yl)methyl]benzonitrile (**57**) | 0.7 | 7.6 | 10.5 |
| 4-[(5-(3-fluorophenyl)-1*H*-imidazol-1-yl)methyl]benzonitrile (**58**) | 5.5 | 31.8 | 5.8 |

| | | | |
|---|---|---|---|
| Ratio = IC₅₀ CYP11B1/ IC₅₀ CYP11B2 | | | |

## Claims

1. The use of a N-benzyl 5-substituted imidazole derivative having the general formula I 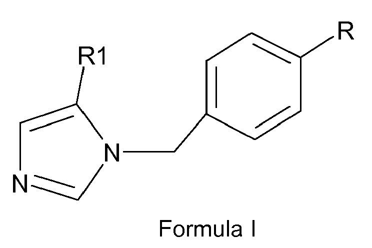 wherein
R is (C₁₋₃)alkyl, (C₁₋₃)alkyloxy, halogen, nitro or cyano;
R₁ is (C₁₋₆)alkyl, optionally substituted with OH, (C₁₋₃)alkyloxy, (C₁₋₃)alkylcarbonyloxy, (C₁₋₃)alkyloxycarbonyl or halogen, or (C₁₋₃)alkyloxycarbonyl; or
R₁ is phenyl, optionally substituted with 1-3 substituents independently selected from (C₁₋₃)alkyl, (C₁₋₃)alkyloxy, hydroxymethyl and halogen; or a pharmaceutically acceptable salt thereof, for the preparation of a medicament for the treatment of a disorder or disease in a subject mediated by aldosterone synthase or responsive to inhibition of aldosterone synthase.

2. The use according to claim 1, wherein the medicament is for the treatment of congestive heart failure and myocardial fibrosis.

3. The use according to claim 1, wherein R is (C₁₋₃)alkyl, (C₁₋₃)alkyloxy, halogen, nitro or cyano, and R₁ is phenyl, optionally substituted optionally substituted with 1-3 substituents independently selected from (C₁₋₃)alkyl, (C₁₋₃)alkyloxy, hydroxymethyl and halogen.

4. The use according to claim 3, wherein R is cyano and R₁ is phenyl, optionally substituted with halogen.

5. The use according to claim 1 wherein the N-benzyl 5-substituted imidazole derivative is selected from:
- 3-[1-(4-fluorobenzyl)-1*H*-imidazol-5-yl]propanol;
- methyl 3-[1-(4-cyanobenzyl)-1*H*-imidazol-5-yl]propanoate;
- methyl 3-[1-(4-bromobenzyl)-1*H*-imidazol-5-yl]propanoate:
- 1-(4-cyanobenzyl)-5-hydroxymethyl-1*H*-imidazole;
- 4-[(5-methyl-1*H*-imidazol-1-yl)methyl]benzonitrile;
- 4-[(5-phenyl-1*H*-imidazol-1-yl)methyl]benzonitrile;
- 4-[(5-formyl-1*H*-imidazol-1-yl)methyl]benzonitrile;
- methyl 1-(4-cyanobenzyl)-1*H*-imidazole-5-carboxylate;
- 1-(4-nitrobenzyl)-5-phenyl-1*H*-imidazole;
- 4-[(5-(2-methylphenyl)-1*H*-imidazol-1-yl)methyl]benzonitrile; and
- 4-[(5-(3-fluorophenyl)-1*H*-imidazol-1-yl)methyl]benzonitrile; or a pharmaceutically acceptable salt thereof.

6. The N-benzyl 5-substituted imidazole derivative according to Figure 1 of claim 1 which is selected from:
- 3-[1-(4-fluorobenzyl)-1*H*-imidazol-5-yl]propanol;
- methyl 3-[1-(4-bromobenzyl)-1*H*-imidazol-5-yl]propanoate;
- 4-[(5-phenyl-1*H*-imidazol-1-yl)methyl]benzonitrile;
- 1-(4-nitrobenzyl)-5-phenyl-1*H*-imidazole;
- 4-[(5-(2-methylphenyl)-1*H*-imidazol-1-yl)methyl]benzonitrile; and
- 4-[(5-(3-fluorophenyl)-1*H*-imidazol-1-yl)methyl]benzonitrile; or a pharmaceutically acceptable salt thereof.

7. A N-benzyl 5-substituted imidazole derivative having the general formula I 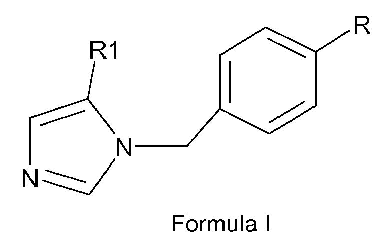 wherein
R is (C₁₋₃)alkyl, (C₁₋₃)alkyloxy, halogen, nitro or cyano;
R₁ is (C₁₋₆)alkyl, optionally substituted with OH, (C₁₋₃)alkyloxy, (C₁₋₃)alkylcarbonyloxy, (Ck₁₋₃)alkyloxycarbonyl or halogen, or (C₁₋₃)alkyloxycarbonyl; or
R₁ is phenyl, optionally substituted with 1-3 substituents independently selected from (C₁₋₃)alkyl, (C₁₋₃)alkyloxy, hydroxymethyl and halogen; or a pharmaceutically acceptable salt thereof, for use in therapy, with the proviso that compounds of formula I wherein R is cyano and R1 is unsubstituted (C₁₋₆)alkyl are excluded.

8. A pharmaceutical composition comprising a N-benzyl 5-substituted imidazole derivative having the general formula I 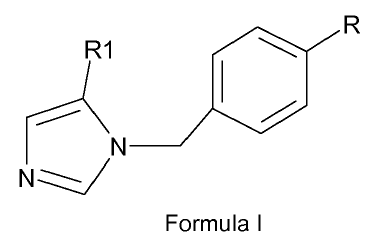 wherein
R is (C₁₋₃)alkyl, (C₁₋₃)alkyloxy, halogen, nitro or cyano;
R₁ is (C₁₋₆)alkyl, optionally substituted with OH, (C₁₋₃)alkyloxy, (C₁₋₃)alkylcarbonyloxy, (C₁₋₃)alkyloxycarbonyl or halogen, or (C₁₋₃)alkyloxycarbonyl; or
R₁ is phenyl, optionally substituted with 1-3 substituents independently selected from (C₁₋₃)alkyl, (C₁₋₃)alkyloxy, hydroxymethyl and halogen; or a pharmaceutically acceptable salt thereof, in admixture with pharmaceutically acceptable auxiliaries, with the proviso that compounds of formula I wherein R is cyano and R₁ is unsubstituted (C₁₋₆)alkyl are excluded.
